# EUROPÄISCHE PATENTSCHRIFT

(11) **EP 0 892 811 B1**
(45) Veröffentlichungstag und Bekanntmachung des Hinweises auf die Patenterteilung: **18.12.2002**
(21) Anmeldenummer: 97918058.5
(22) Anmeldetag: 26.03.1997
(51) Int. Cl.: C07K 14/00

(54) **PEPTIDE MIT ANTIPROLIFERATIVEN EIGENSCHAFTEN**
PEPTIDES WITH ANTIPROLIFERATIVE PROPERTIES
PEPTIDE PRESENTANT DES PROPRIETES ANTIPROLIFERATIVES

(30) Priorität: 26.03.1996 DE 19611939; 20.12.1996 DE 19653445
(43) Veröffentlichungstag der Anmeldung: 27.01.1999
(60) Teilanmeldung: 98118811.3
(73) Patentinhaber: Radulescu, Razvan T., 81375 München (DE)
(72) Erfinder: Radulescu, Razvan T., 81375 München (DE)
(74) Vertreter: Becker Kurig Straus
(86) Internationale Anmeldenummer: DE9700643
(87) Internationale Veröffentlichungsnummer: WO97035873

(56) Entgegenhaltungen:
- EP-A- 0 666 270
- EP-A- 0 685 493
- WO-A-89/06703
- WO-A-94/25477
- WO-A-95/34647
- WO-A-97/04006
- DE-A- 4 237 129
- RADULESCU R T ET AL: "Binding of the LXCXE insulin motif to a hexapeptide derived from retinoblastoma protein" BIOCHEMICAL AND BIOPHYSICAL RESEARCH COMMUNICATIONS., Bd. 206, Nr. 1, 5.Januar 1995, ORLANDO, FL US, Seiten 97-102, XP002045809 in der Anmeldung erwähnt
- WENDTNER C M ET AL: "Prediction of homologous binding sites on RB and P107 common for viral oncoproteins and cellular ligands" JOURNAL OF MOLECULAR RECOGNITION, Bd. 5, Nr. 4, 1992, LONDON GB, Seiten 125-132, XP002045810
- RADULESCU R T : "The 'LXCXE' hydropathic superfamily of ligands for retinoblastoma protein: A proposal" MEDICAL HYPOTHESES, Bd. 44, Nr. 1, 1995, MONTREAL CA, Seiten 28-31, XP002045811

## Beschreibung

Die vorliegende Erfindung betrifft Peptide mit antiproliferativen Eigenschaften, die sie kodierenden Nukleinsäuren (DNAs/RNAs), zu diesen Nukleinsäuren strukturhomologe Peptidnukleinsäuren sowie die Verwendung der Peptide, der Nukleinsäuren, der Peptidnukleinsäuren und/oder ihrer pharmazeutischen Zusammensetzung in der Biotechnologie, der Molekularbiologie, der Bioinformatik und der Diagnose und Therapie von hyperproliferativen Erkrankungen, insbesondere von Krebs und der Atherosklerose.

Im Bereich der Onkologie gibt es derzeit zahlreiche feststehende Behandlungsschemata, die vor allem auf Operationsverfahren, Chemo- und Strahlentherapie basieren. Allerdings haben diese Methoden noch nicht den gewünschten Durchbruch in der Krebstherapie gebracht. Laut einer aktuellen amerikanischen Statistik erkrankt jeder dritte lebende Amerikaner an Krebs und jeder fünfte stirbt daran. Diese Zahl dürfte für die Industrienationen weltweit zutreffen und ist schon seit mehr als einem Jahrzehnt relativ unverändert. Weiterhin ist festzuhalten, daß sich seit zwanzig Jahren die Effizienz der Krebsbehandlung nicht deutlich verbessert hat, da die Fünf-Jahres-Überlebensraten für die Mehrzahl der Krebsarten ungefähr gleich geblieben sind (R.N. Proctor "The Sciences", März/April 1995, pp. 20-24).

Die Aufgabe der vorliegenden Erfindung besteht deshalb darin, eine Möglichkeit bereitzustellen, mit der das Wachstum vieler verschiedener Krebsarten effizient gebremst werden kann und es zu einer Rückbildung der Tumore kommt.

Gelöst wird diese Aufgabe durch ein Peptid gemäß Anspruch 1, durch eine Peptidnukleinsäure gemäß Anspruch 10, durch eine pharmazeutische Zusammensetzung gemäß Anspruch 13 sowie durch die Verwendung der Peptide gemäß Anspruch 16, die Verwendung einer Nukleinsäure gemäß Anspruch 28, die Verwendung der Peptidnukleinsäure gemäß Anspruch 31 und die Verwendung der pharmazeutischen Zusammensetzung gemäß Anspruch 32. Vorteilhafte Ausgestaltungen ergeben sich aus den Unteransprüchen.

Von dem Erfinder wurde gefunden, daß ein vielversprechender Ansatz in der Behandlung von Krebs darin besteht, spezifische Peptide einzusetzen, die krebsverursachende Substanzen, insbesondere onkogene Proteine blockieren. Die erfindungsgemäßen Peptide zeichnen sich dadurch aus, daß sie eine geringe Sequenzlänge haben und damit vom Synthesestandpunkt her gesehen ökonomisch sind. Außerdem können sie effizient intrazellulär penetrieren, um damit die innerhalb der Zelle lokalisierten, den Krebsprozeß unmittelbar vorantreibender Zielproteine zu neutralisieren.

Grundlage der Erfindung ist die Erkenntnis, daß bei vielen Krebsarten Tumorsuppressorgene deletiert oder mutiert sind, so daß deren Genprodukte nicht in ausreichender Menge vorhanden sind, was zur Krebsentwicklung führt (A.G. Knudson, Proc. Natl. Acad. Sci., USA, 1993, Vol. 90, pp. 10914-10921; A.J Levine, Sci. Am. "Science & Medicine", Jan./Febr. 1995, pp. 28-37). Bei anderen hyperproliferativen Erkrankungen wie z.B. der Atherosklerose scheint ebenfalls ein Defekt von Tumorsuppressorproteinen, z.B. von RB1 pathogenetisch von Bedeutung zu sein (Chang, M.W. et al. Science 1995 Vol. 267, pp. 518-522). Vom Erfinder wurde nun gefunden, daß es genügt, Teile eines Tumorsuppressorproteins in die Zelle einzuführen, um Wachstumsfaktoren, die das unkontrollierte Zellwachstum beschleunigen, zu hemmen. Es ist nämlich zu umständlich das gesamte Tumorsuppressorprotein herzustellen und in die Zelle einzu bringen, da ein solches Protein zu lang ist und meist einer proteolytischen Spaltung unterliegt, bevor es seine Wirkung entfalten kann. Verwendet man andererseits nur Teile aus dem Tumorsuppressorprotein werden auch diese zu schnell abgebaut, d.h. sie sind nicht ausreichend stabil.

Bei den erfindungsgemäßen Peptiden handelt es sich deshalb um synthetische Fusionspolypeptide aus den Bausteinen (A) und (B), wobei diese Bausteine in Kombination von AB oder BA vorliegen können. Der erste Baustein (A) enthält einen wirksamen Abschnitt eines Tumorsuppressorproteins - so wie vom Erfinder erstmalig herausgefunden - bzw. ist dazu hydropathisch homolog. Der zweite Teil (B) weist eine Sequenz auf, die den ersten Teil (A) stabilisiert. Erfindungsgemäß bindet das erfindungsgemäße Peptid an einen Wachstumsfaktor oder Wachstumsfaktor-Rezeptor, bevorzugt an eine LXCXE-Sequenz darin, vor allem an die LXCXE-Sequenz in Insulin und zwar an LVCGE (Radulescu et al., Biochemical and Biophysical Research Communications 1995, Vol. 206, pp. 97-102). Da die Wachstumsfaktoren IGF-1 und IGF-2 die Sequenz FVCGD beherbergen, die hydropatisch homolog zum Abschnitt LVCGE in Insulin ist (R. Radulescu & C. Wendtner, J. Mol. Recognition 1992, Vol 5, pp. 133-137), bindet das erfindungsgemäße Peptid auch an IGF-1 und IGF-2.

Der oben verwendete Begriff "hydropathisch homolog" wird im Sinne der Lehren von Kyte und Doolittle (J. Kyte & R.F. Doolittle, J. Mol. Biol. 1982, Vol. 157, pp. 105-132) und J.E. Blalock und Smith, E.M. (Biochemical and Biophysical Research Communications, Vol. 121, No. 1, S. 203-207 (1984)) verwendet.

Weiter zeichnet sich der Teil (A) des erfindungsgemäßen Peptids dadurch aus, daß er im Sinne der Komplementärpeptid-Theorie (J.E. Blalock, Trends in Biotechnology. Vol. 8, pp. 140-144, Juni 1990) bzw. im Sinne der dreidimensionalen Konfiguration zu einem Fragment eines Wachstumsfaktors oder Wachstumsfaktor-Rezeptors hydropathisch komplementär ist. Beispiele für Wachstumsfaktoren sind: Insulin, IGF-1, IGF-2, EGF, FGF, Angiogenin, NGF und PDGF.

Erfindungsgemäß können die für Teil (A) des erfindungsgemäßen Peptids in Frage kommenden wirksamen Abschnitte aus den folgenden Tumorsuppressorgen-Produkten stammen: RB1, P107, P130, WT1, TP53, NF1, NF2, VHL, APC, NB1, MLM, MEN1, BCNS, RCC, BRCA1, BRCA2, DCC, MTS1=p16, MTS 2, P21, P27 und anderen. Bevorzugt umfaßt der Teil (A) einen Abschnitt aus RB1 und zwar die Aminosäuren 649-654 von RB1, d.h.: LFYKKV bzw. ist hydropathisch homolog dazu.

Der Teil (B) des Fusionspeptids sorgt als Kofaktor dafür, daß der Teil (A) stabilisiert wird, insbesondere daß es zu keinem proteolytischen Abbau in der Zelle kommt. Dies kann einerseits dadurch geschehen, daß die erfindungsgemäßen Peptide schnell in den Zellkern hineingelangen und dort gegenüber Proteasen weniger anfällig sind (R. Fahraeus et al., Current Biology 1996, Vol. 6, No. 1, pp 84-91)oder indem bei verzweigten Teilen (B) z.B. Polylysin-Verzweigungen bzw.- Core S oder D-Aminosäuren verwendet werden. In bevorzugter Weise ist der Teil (B) ein Polylysin-Core (R. Radulescu et al., Biochemical and Biophysical Research Communications, 1995, Vol. 206, pp. 97-102 sowie G. Fassina, EPO 0 481 930 A2) oder eine nukleäre Lokalisationssequenz (NLS), insbesondere eine SV40-NLS oder Penetratin oder eine bipartite NLS oder die RNP A1 NLS (M9 Region). Allgemeine Hinweise für die Konstruktion von Peptiden, die bevorzugt in den Zellkern hineingelangen, ergeben sich aus Sheldon et al., Proc. Natl., Acad. Sci. USA, Vol. 92, S. 2056-2060 (1995); Dingwall et al., Trends in Biochemical Sciences 16, S. 478-481 (1991) M.S. Moore, Current Biology, Vol. 6, No. 2, S. 137-140 (1996); D.A. Jans, FASEB Journal, 1994, Vol. 8, pp. 841-847; J. Moroianu & J.F. Riordan PNAS, 1994, Vol. 91, pp. 1677-1681 und D. Derossi et al., Journal of Biological Chemistry, 1994, Vol. 269, pp. 10444-10450.

Bevorzugt hat der Teil (A) folgende Sequenz bzw. ist dazu in mindestens zwei Positionen hydropathisch homolog:

NH₂-L-F-Y-K-K-V-COOH (P1)

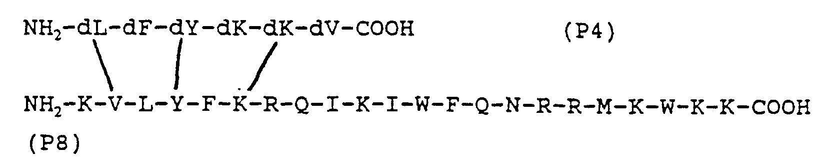

### (Striche kennzeichnen eine hydropathische Homologie)

Daraus ergeben sich folgende bevorzugte erfindungsgemäße Peptide:

NH₂-[L-F-Y-K-K-V-GGG]₄-[K-R-G]₂-K-G-COOH (P2)

Dieses Peptid ist eine Kombination aus der Sequenz (P1) und dem Polylysin-Core [-GGG]₄-[K-R-G]₂-K-G.

NH₂-[L-F-Y-K-K-V-GGG ]₄-[K]₂-K-G-COOH (P2i)

Dieses Peptid ist eine Kombination aus der Sequenz P1 und dem Polylysin-Core [-GGG]₄-[K]₂-K-G.

NH₂-[dL-dF-dY-dK-dK-dV-GGG]₄(lK-dR-G]₂-lK-G-COOH (P3)

Dieses Peptid ist eine Kombination aus der Sequenz (P4) und dem Polylysin-Core

[-GGG]₄-[1K-dR-G]₂-1K-G.

NH₂-[dL-dF-dY-dK-dK-dV-GGG]₄-[lK]₂-lK-G-COOH (P3i)

Dieses Peptid ist eine Kombination aus der Sequenz (P4) und dem Polylysin-Core [-GGG]₄-[lK]₂-1K-G.

NH₂-L-F-Y-K-K-V-P-K-K-K-R-K-V-COOH (P5)

Dieses Peptid (P5) setzt sich zusammen aus der Sequenz (P1) und der nukleären Lokalisationssequenz des Large T Antigen des SV40-Virus.

NH₂-L-F-Y-K-K-V-R-Q-I-K-I-W-F-Q-N-R-R-M-K-W-K-K-COOH (P6)

Dieses Peptid (P6) ist eine Kombination aus der Sequenz (P1) und Penetratin, einer 16 Aminosäuren langen Sequenz innerhalb der Antennapedia-Homeodomäne, die der Membrantranslokation und somit auch der nuklearen Lokalisation dient (s.o.).

NH₂-[dK-dV-dL-dY-dF-dK-GGG]₄-[lK-dR-G]₂-lK-G-COOH (P7s)

Dieses Peptid (P7s) ist eine Kombination aus der Sequenz dK-dV-dL-dY-dF-dK, die hydropathisch homolog ist zu P1 bzw. P4 und dem Polylysin-Core [-GGG]₄-[lK-dR-G]₂-lK-G.

NH₂-[dK-dV-dL-dY-dF-dK-GGG]₄-[lK]₂-lK-G-COOH (P7)

Dieses Peptid (P7) ist eine Kombination aus der Sequenz dK-dV-dL-dY-dF-dK, die hydropathisch homolog ist zu P1 und dem Polylysin-Core [-GGG]₄-[lK]₂-lK-G.

NH₂-K-V-L-Y-F-K-R-Q-I-K-I-W-F-Q-N-R-R-M-K-W-K-K-COOH (P8)

Dieses Peptid (P8) ist eine Kombination aus der Sequenz K-V-L-Y-F-K, die hydropathisch homolog ist zu P1 und aus Penetratin.

"d" und "1" bezeichnen die Konfiguration der im Ein-Buchstaben-Code dargestellten Aminosäuren.

Allgemein weisen die erfindungsgemäßen Peptide L-und/oder D-Aminosäuren auf. Erfindungsgemäß sollen all-L-Formen, all-D-Formen, die retro-inverso-Isomere sowie die entsprechenden Permutationen von L- und D-Form jeder einzelnen Aminosäure möglich sein. Darüberhinaus sollen erfindungsgemäß alle Permutationen von oxidierter und reduzierter Form jeder einzelnen Aminosäure sowie von freier und Schutzgruppe(n) tragender Aminosäure möglich sein. Die optimalen Wirkkonzentrationen der erfindungsgemäßen Peptide sind erfindungsgemäß im Bereich von 10⁻⁴M bis 10⁻⁵M, andere optimale Wirkkonzentrationsbereiche sind allerdings je nach Anwendungsbereich erfindungsgemäß auch möglich. Für verzweigte Peptide hat es sich als vorteilhaft herausgestellt, daß sie in der all-D-Form sind. Lineare, eine NLS enthaltende Peptide gehen höchstwahrscheinlich in den Zellkern hinein; verzweigte Peptide gehen wahrscheinlich in den Zellkern, können aber auch außerhalb davon ihre Wirkung entfalten.

Die erfindungsgemäßen Peptide kann man als "SCAPs" bezeichnen, d.h. "Synthetische Cofaktor-verbundene antionkogene Peptide".

Die Herstellung der erfindungsgemäßen Peptide erfolgt bevorzugt synthetisch nach der üblichen Solid-Phase-Methode (siehe G.A. Grant, "Synthetic Peptides", W.H. Freeman and Company, New York, 1992). Die anschließende Reinigung der erfindungsgemäßen Peptide wurde durchgführt und überprüft wie schon beschrieben (R. Radulescu et al., Biochemical and Biophysical Research Communications, 1995, Vol. 206, pp. 97-102). Letztere Literaturangabe liefert auch ein Beispiel, wie man die erfindungsgemäßen Peptide zur biotechnologischen Reingewinnung von Wachstumsfaktoren, z.B. von Insulin, und Wachstumsfaktor-Rezeptoren einsetzen kann.

Die eine NLS tragenden erfindungsgemäßen Peptide könnte man auch an eine Heparansulfatmatrix tragende Chromatographiesäule koppeln zwecks biotechnologischer Reingewinnung von Wachstumsfaktoren und Wachstumsfaktor-Rezeptoren.

Die Teile (A) und (B) der erfindungsgemäßen Peptide können allerdings auch nach Standardmethoden aus den entsprechenden Proteinen herausgespalten werden und miteinander verbunden werden. Weitere Techniken zur Herstellung der Peptide sind dem Fachmann geläufig (siehe G.A. Grant, "Synthetic Peptides", W.H. Freeman and Company, New York, 1992).

Die Erfindung betrifft weiter eine DNA/RNA kodierend für die erfindungsgemäßen Peptide, wobei die DNA/-RNA-Sequenz über den genetischen Code abgeleitet werden kann.

In bevorzugter Weise kodiert die folgende DNA/RNA für die oben angegebenen Aminosäurensequenzen (P1) und (P4) des Teils (A) eines erfindungsgemäßen Peptids:

5'-CUU UUC UAC AAG AAG GUU-3' (D1)

In bevorzugter Weise kodiert die folgende DNA/RNA für das oben angegebene erfindungsgemäße Peptid (P5):

5′-CUU UUC UAC AAG AAG GUU CCU AAG AAG AAG CGU AAG
GUU-3′ (D5)

In bevorzugter Weise kodiert die folgende DNA/RNA für das oben angegebene erfindungsgemäße Peptid (P6):

5′-CUU UUC UAC AAG AAG GUU CGU CAA AUA AAG AUA UGG
UUC CAA AAU CGU CGU AUG AAG UGG AAG AAG-3′ (D6)

In bevorzugter Weise kodiert die folgende DNA/RNA für das oben angegebene erfindungsgemäße Peptid (P8):

5'-AAG GUU CUU UAC UUC AAG CGU CAA AUA AAG AUA UGG
UUC CAA AAU CGU CGU AUG AAG UGG AAG AAG-3'   (D8)

Die für die erfindungsgemäßen Peptide kodierenden DNAs/RNAs können auch in entsprechende Vektoren eingebaut werden zwecks Einsatz in der Gentherapie gegen Krebs. Eine Übersicht der Methodologie findet sich bei R.C. Mulligan, Science 1993, Vol. 260, pp. 926-932.

Die oben angegebenen DNA/RNA-Sequenzen schließen auch damit unter stringenten Bedingungen, wie sie dem Fachmann geläufig sind, hybridisierende DNAs/RNAs ein. Insbesondere DNAs/RNAs, die bei etwa 20 °C unter dem Schmelzpunkt der DNA/RNA mit den oben angegebenen DNAs/RNAs hybridisieren. Außerdem schließen die oben angegebenen DNA/RNA-Sequenzen DNAs/RNAs ein, die mit den oben angegebenen DNA/RNA-Sequenzen DNAs/RNAs über den degenerierten genetischen Code verwandt sind.

Eine weitere Anwendung der Erfindung ergibt sich für die Bioinformatik und Molekularbiologie. Um Tumorsuppressorproteine zu identifizieren, die mit einem Wachstumsfaktor oder dessen Rezeptor interagieren, könnte man folgende Strategie anwenden. Die cDNA des betreffenden Wachstumsfaktors bzw. dessen Rezeptors könnte man aus der NCBI-Datenbank abrufen und im Sinne der Komplementärpeptidstrategie diese cDNA in eine komplementäre DNA umschreiben und dann in ein Peptid übersetzen mit Hilfe der DNA Strider Software. Für die sich daraus ergebenden Komplementärpeptide könnte man homologe Proteine/Peptide (die gesuchten Tumorsuppressoren) mit Hilfe des BLAST-Algorithmus im OWL-Datenbanknavigator finden. Umgekehrt könnte man auch vorgehen, in dem man von einer Tumorsuppressor-cDNA ausgeht und analog zum obigen Vorgehen dafür Wachstumsfaktoren bzw. deren Rezeptoren als Interaktionspartner ausfinding macht. Dieses Verfahren könnte auch das Klonieren von (neuen) Tumorsuppressorgenen oder (neuen) Wachstumsfaktor- bzw. Wachstumsfaktor-Rezeptor-Genen wesentlich beschleunigen und erleichtern. Beispiel: Die cDNA des humanen EGF-Vorläufer-Proteins wurde aus der NCBI-Datenbank herauskopiert und in die DNA Strider Software hineinkopiert, wo mit Hilfe dieser Software die komplementäre(n) DNA(s) zu der EGF-Vorläufer-cDNA abgeleitet wurden, und diese DNA(s) in Peptide übersetzt wurden, in sogenannte Komplementärpeptide zum EGF-Vorläufer-Protein. In einem weiteren Schritt wurde nach homologen Peptiden/-Proteinen zu diesen Komplementärpeptiden mit Hilfe des BLAST-Algorithmus im OWL-Datenbanknavigator gesucht. Das Ergebnis dieser Suche war, daß mehrere nukleäre Proteine zum EGF-Vorläufer Komplementär waren, darunter das mit RB1 strukturell und funktionell verwandte p130-Protein, genauer die p130-Aminosäuren 290-313. Noch genauer: Die EGF-Vorläufer-Aminosäuren 209-213 (REGSN) und die p130-Aminosäuren 305-309 (IGTLS) sind hydropathisch komplementär zueinander und damit potentielle Bindungsstellen in der möglichen Komplexbildung zwischen dem EGF-Vorläufer und p130, wenn man diese Regionen antiparallel zueinander anordnet. Damit könnte die Aminosäuresequenz IGTLS bzw. hydropathisch homologe Sequenzen dazu als Teil (A) eines antiproliferativen Peptids im Sinne der vorliegenden Erfindung dienen (s. Anspruch 1 u. 2).

Für die therapeutische Anwendung werden die erfindungsgemäßen Peptide, einzeln oder in Kombination von mehreren, üblicherweise mit Hilfs-, Füll- und/oder Zusatzstoffen zusammen in einer pharmazeutischen Zusammensetzung verwendet. Besonders vorteilhaft hat sich die Kombination der oben angegebenen Peptide P3 und P6 bzw. P3 und P5 herausgestellt. Als Darreichungsformen der pharmazeutischen Zusammensetzung kommen dabei in Frage: Salben, Lösungen, Dispersionen, Emulsionen, Aerosole, Schäume, teilchenförmige Mittel (z.B. Granulate, Agglomerate, Puder, Mikroperlen, Adsorbate), Pillen, Pastillen, Tabletten, Dragees oder Kapseln. Die erfindungsgemäßen Peptide können auch in Kombination mit anderen Zytostatika oder in Kombination mit Bestrahlungsmethoden verwendet werden.

Die Verabreichung erfolgt vorzugsweise lokal, intracutan oder transcutan; zur systemischen Anwendung vorzugsweise intravenös, intraarteriell, oral, rectal; zur Anwendung in Hohlräumen vorzugsweise intrathekal, intraperitoneal oder intracavitär.

Die erfindungsgemäßen Peptide und DNAs/RNAs sowie die erfindungsgemäße pharmazeutische Zusammensetzung eignen sich als Krebstherapeutika und werden erfindungsgemäß in dieser Weise verwendet, wobei sich ein ausgeprägter zytotoxischer Effekt zeigt.

Bevorzugte Wirksamkeit besteht gegen Mammakarzinom-, Osteosarkom- und Leukämiezellen. Aufgrund seiner Konzeption wirkt das erfindungsgemäße Peptid allgemein gegen alle Tumorzellen, die ein defektes Retinoblastomgen bzw. -protein aufweisen.

Die Erfindung wird weiter anhand der 15 Figuren und 2 Tabellen beschrieben.
- Fig. 1 bis Fig. 12:: Auftragung der % Zellpopulationen in der G1-, S- und G2/M-Phase von MCF-7- oder SAOS-2-Zellen und in der An- oder Abwesenheit verschiedener erfindungsgemäßer Peptide
- Fig. 13:: Zytotoxischer Effekt des P3-Peptids auf K562-Zellen
- Fig. 14:: Zytotoxischer Effekt des P3-Peptids auf CCRF-CEM und CCRF-CEM/ACT 400 - Zellen
- Fig. 15:: Effekt des P3-Peptids auf normale periphere mononukleäre Blutzellen
- Tab 1:: Bremsen der durch IGF-1 bzw. Insulin induzierten Zellzyklusprogression von MCF-7-Zellen durch P5 und P6, nicht aber durch Penetratin
- Tab 2:: Bremsen der durch FCS induzierten Zellzyklusprogression von SAOS-2-Zellen durch P5 und P6, nicht aber durch Penetratin

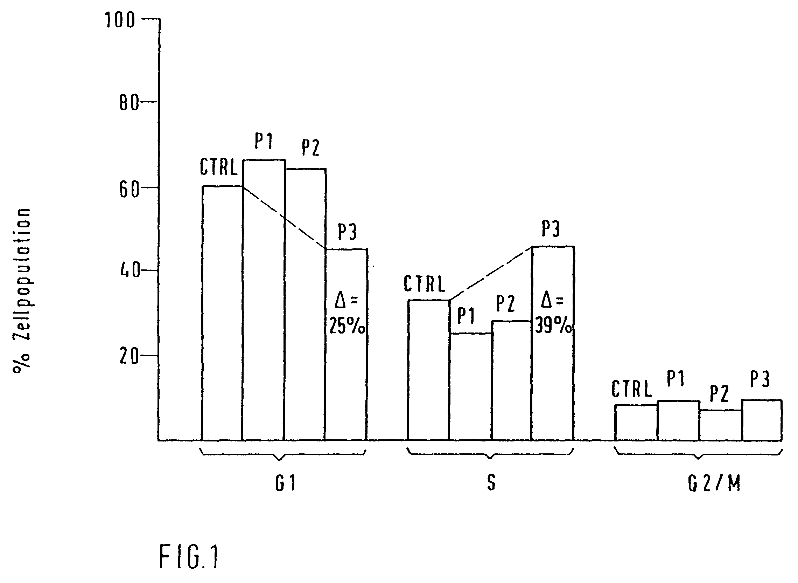

Fig. 1 zeigt, daß das erfindungsgemäße Peptid P3[10⁻⁵M] die G1-Phase vermindert und die S-Phase in der G0/G1-Phase synchronisierter MCF-7-Zellen unter serumfreien Bedingungen erhöht. Die Morphologie der mit P3 behandelten Zellen entspricht der apoptotischer Zellen.

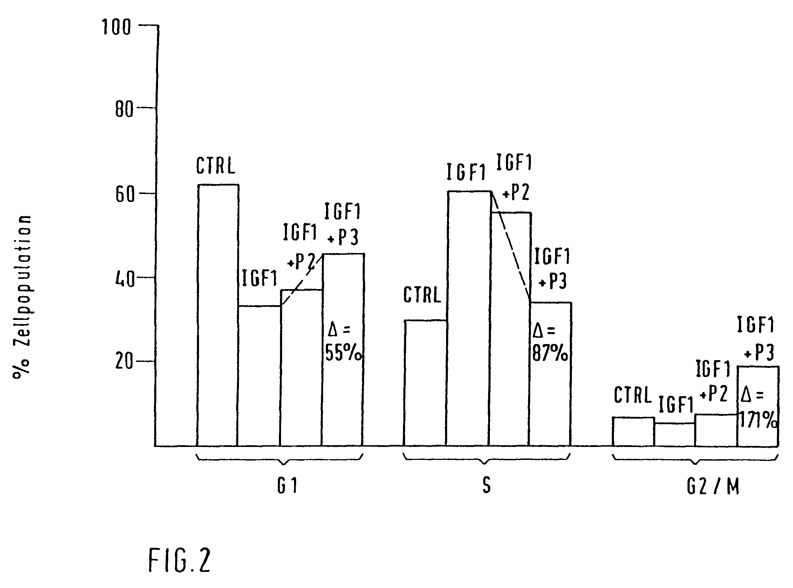

Fig. 2 zeigt, daß das erfindungsgemäße Peptid P3 [10⁻⁵M] zu einer Zunahme der G1- und G2/M-Phasen sowie zu einer Abnahme der S-Phase von MCF-7-Zellen, die durch insulin-like growth factor 1, kurz IGF-1, in einer optimalen Konzentration von [10⁻⁸M] stimuliert wurden, führt. P3 blockiert damit den Effekt von IGF-1 auf MCF-7 Zellen, genauer: das durch IGF-1 bedingte Fortschreiten des Zellzyklus und damit die Zellteilung wird durch P3 gebremst. Die Morphologie der mit P3 behandelten Zellen entspricht der apoptotischer Zellen.

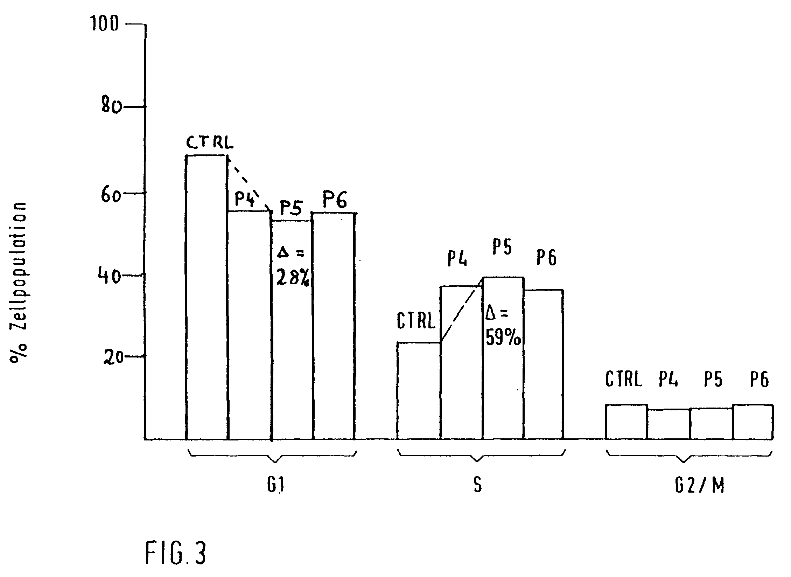

Fig. 3 zeigt, daß jedes der erfindungsgemäßen Peptide P4, P5 und P6 - jeweils in einer Konzentration von 10⁻⁵M - die G1-Phase vermindert und die S-Phase in der G0/G1-Phase synchronisierter MCF-7-Zellen unter serumfreien Bedingungen steigert. Die Morphologie insbesondere der mit P6 behandelten Zellen entspricht der apoptotischer Zellen.

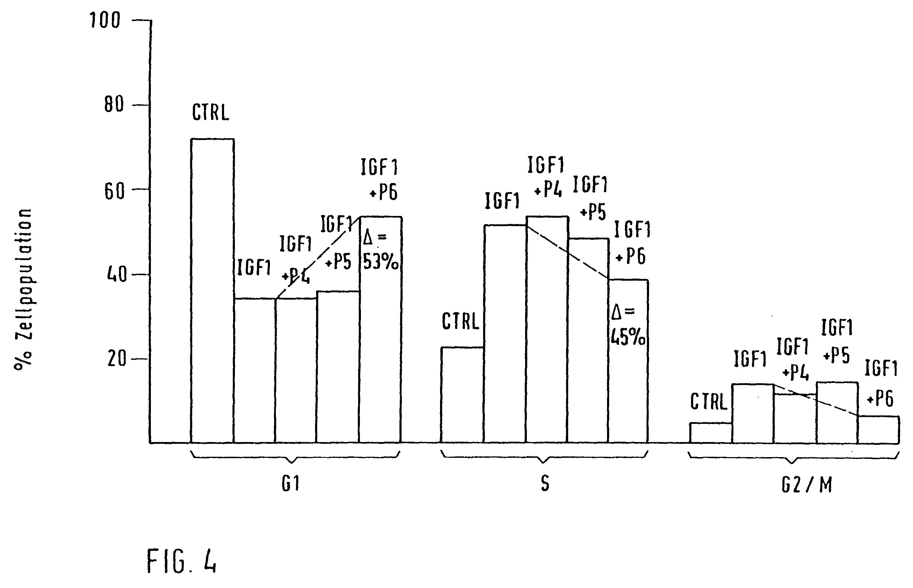

Fig. 4 zeigt, daß das erfindungsgemäße Peptid P6 [10⁻⁵M] zu einer Zunahme der G1-Phase sowie einer Abnahme der S-Phase von MCF-7-Zellen führt, die durch IGF-1 in einer optimalen Konzentration von [10⁻⁸M] stimuliert wurden. P6 blockiert damit den Effekt von IGF-1 auf MCF-7 Zellen, genauer: das durch IGF-1 bedingte Fortschreiten des Zellzyklus und damit die Zellteilung wird durch P6 gebremst. Die Morphologie der mit P6 behandelten Zellen entspricht der apoptotischer Zellen.

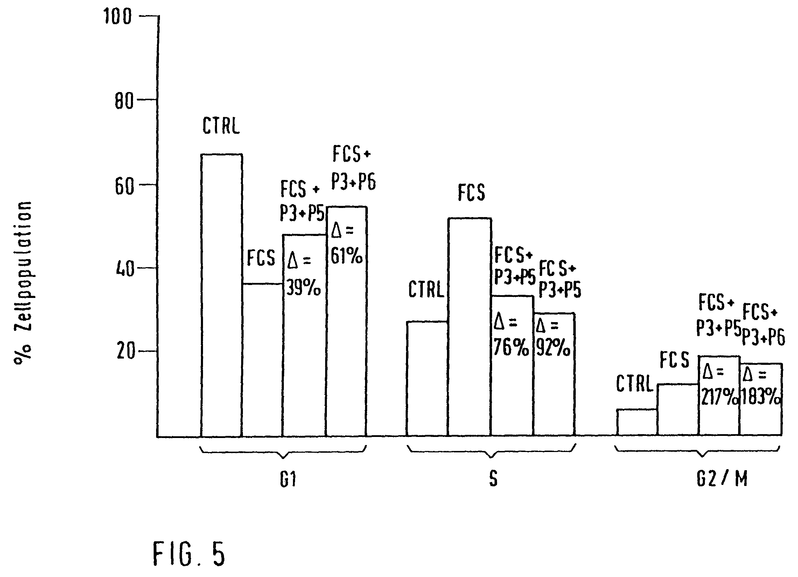

Fig. 5 zeigt, daß die Kombination der erfindungsgemäßen Peptide P3 [10⁻⁵M] und P5 [10⁻⁵M] sowie die Kombination der erfindungsgemäßen Peptide P3 [10⁻⁵M] und P6 [10⁻⁵M] jeweils zu einer Zunahme der G1 und G2/M-Phasen sowie einer Abnahme der S-Phase von MCF-7-Zellen führt, die durch 10 % Fetales Kalbsserum (kurz: FCS) stimuliert wurden. Die Morphologie der mit diesen Peptidkombinationen behandelten Zellen entspricht jeweils der apoptotischer Zellen.

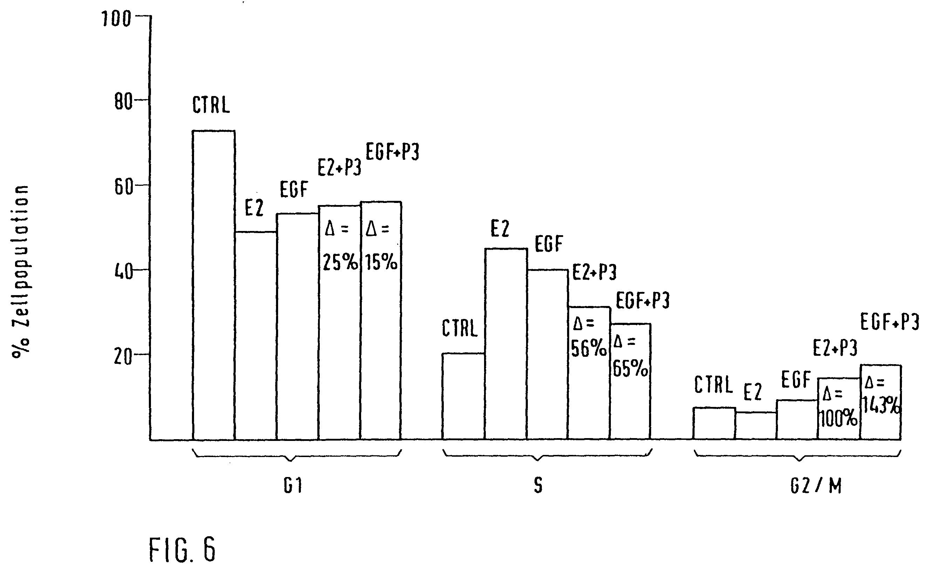

Fig. 6 zeigt, daß das erfindungsgemäße Peptid P3 [10⁻⁵M] zu einer Zunahme der G1- und G2-Phasen sowie ei-ner Abnahme der S-Phase von MCF-7-Zellen führt, die durch östradiol, kurz E2, in einer optimalen Konzentration von [10⁻⁹M] bzw. durch epidermal growth factor, kurz: EGF, in einer optimalen Konzentration von [10⁻⁸M] stimuliert wurden. Die Morphologie der mit P3 behandelten Zellen entspricht der apoptotischer Zellen.

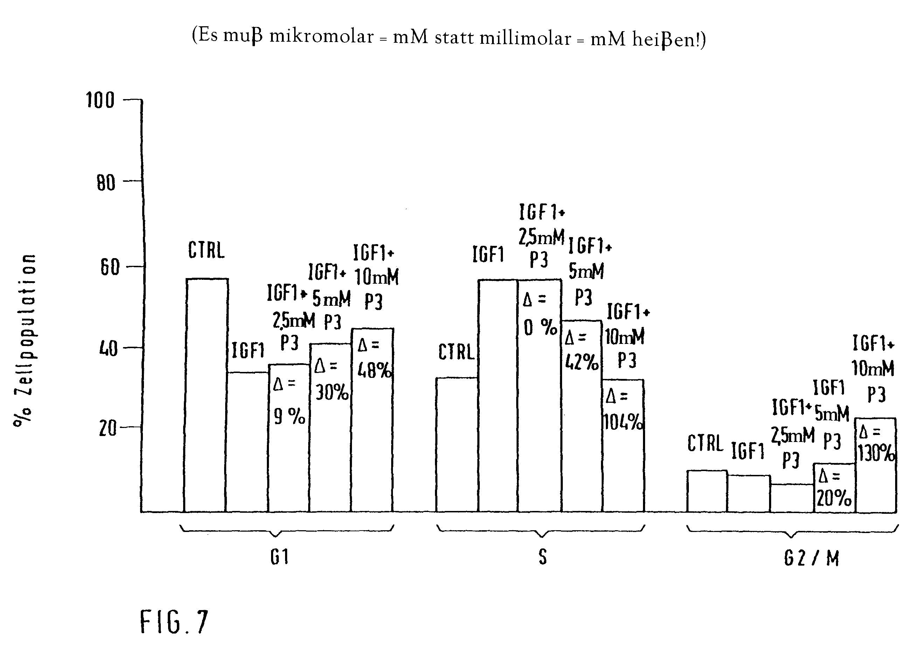

Fig. 7 zeigt, daß das erfindungsgemäße Peptid P3 das durch IGF-1 [10⁻⁸M] bedingte Fortschreiten des Zellzyklus in einer konzentrationsabhängigen Weise blockiert. Die Morphologie der mit P3 [5 x 10⁻⁶M] und insbesondere mit P3 [10⁻⁵M] behandelten Zellen entspricht jeweils der apoptotischer Zellen.

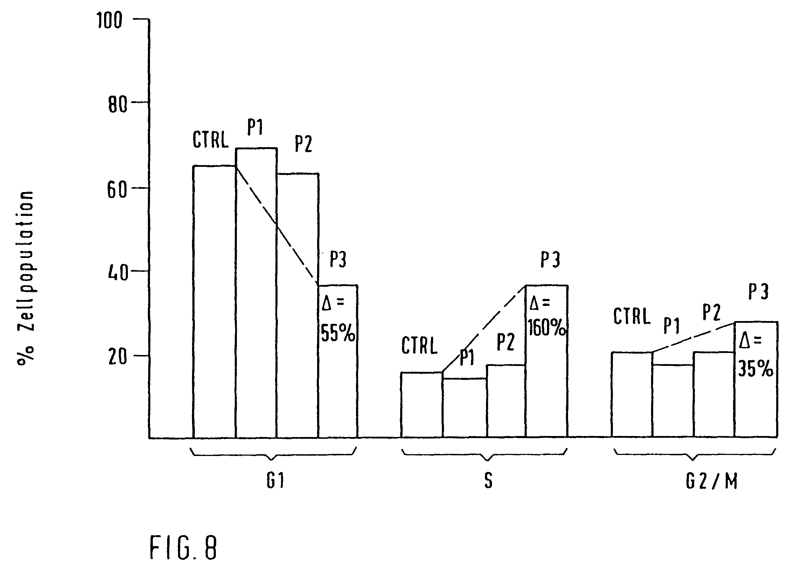

Fig. 8, macht deutlich, daß das erfindungsgemäße Peptid P3 [10⁻⁵M] die G1-Phase vermindert und die S- und G2-Phasen in der G0/G1-Phase synchronisierter SAOS-2-Zellen unter serumfreien Bedingungen erhöht. Die Morphologie der mit P3 behandelten Zellen entspricht der apoptotischer Zellen.

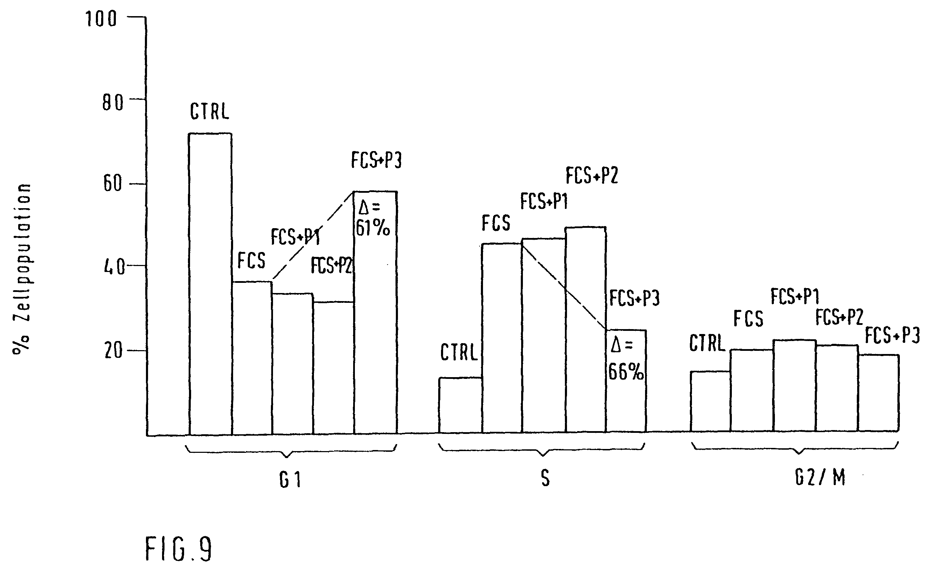

Fig. 9 zeigt, daß das erfindungsgemäße Peptid P3 [10⁻⁵M] zu einer Zunahme der G1-Phase sowie einer Abnahme der S-Phase von SAOS-2-Zellen führt, die durch 10 % FCS stimuliert wurden. Die Morphologie der mit P3 behandelten Zellen entspricht der apoptotischer Zellen.

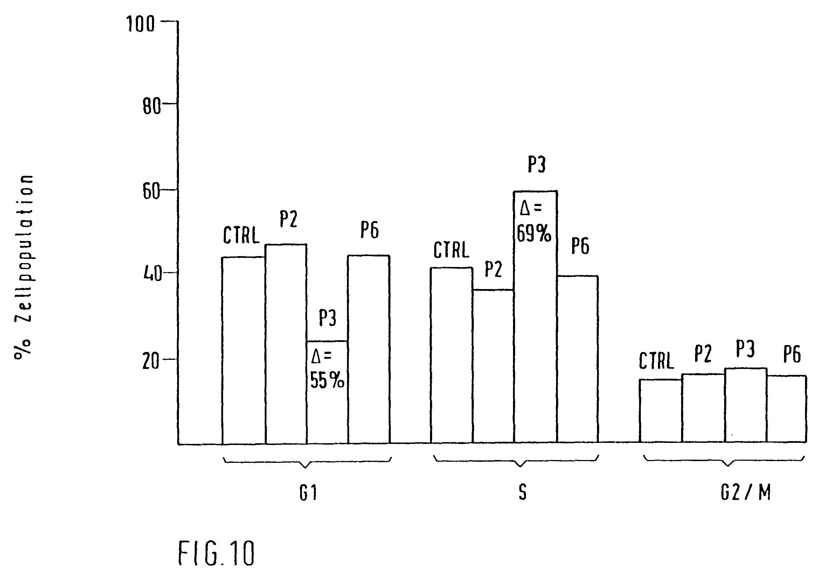

Fig. 10 zeigt, daß das erfindungsgemäße Peptid P3 [10⁻⁵M] die G1-Phase vermindert und die S-Phase von *asynchronen* MCF-7-Zellen erhöht, die in DMEM-Zellkulturmedium mit 10 % FCS inkubiert wurden. Die Morphologie der mit P3 behandelten Zellen entspricht der apoptotischer Zellen.

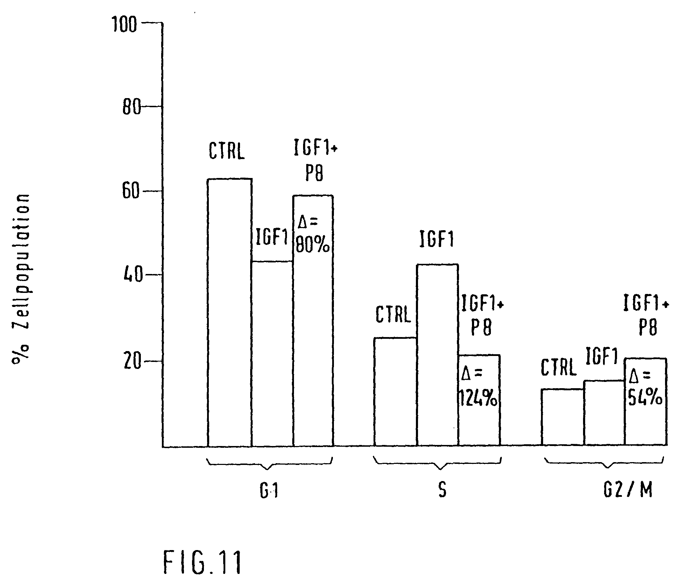

Fig. 11 zeigt, daß das erfindungsgemäße Peptid P8 [10⁻⁵M] zu einer Zunahme der G1- und G2/M-Phasen sowie einer Abnahme der S-Phase von MCF-7-Zellen führt, die durch IGF-1 in einer optimalen Konzentration von [10⁻⁸M] stimuliert wurden. P8 blockiert damit den Effekt von IGF-1 auf MCF-7-Zellen, genauer: das durch IGF-1 bedingte Fortschreiten des Zellzyklus und damit die Zellteilung wird durch P8 gebremst. Die Morphologie der mit P8 behandelten Zellen entspricht der apoptotischer Zellen.

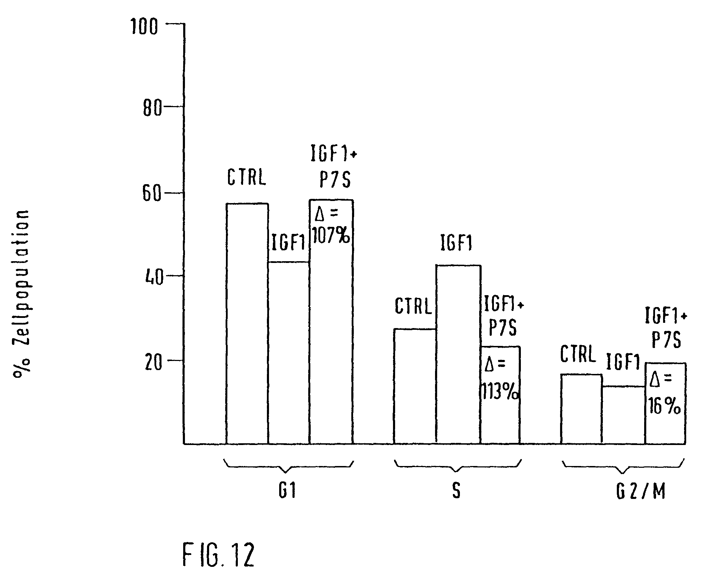

Fig. 12 zeigt, daß das erfindungsgemäße Peptid P7s [10⁻⁵M] zu einer Zunahme der G1- und G2-/M-Phasen sowie einer Abnahme der S-Phase von MCF-7-Zellen führt, die durch IGF-1 in einer optimalen Konzentration von [10⁻⁸M] stimuliert wurden. P7s blockiert damit den Effekt von IGF-1 auf MCF-7-Zellen, genauer: das durch IGF-1 bedingte Fortschreiten des Zellzyklus und damit die Zellteilung wird durch P7s gebremst. Die Morphologie der mit P7s behandelten Zellen entspricht der apoptotischer Zellen.

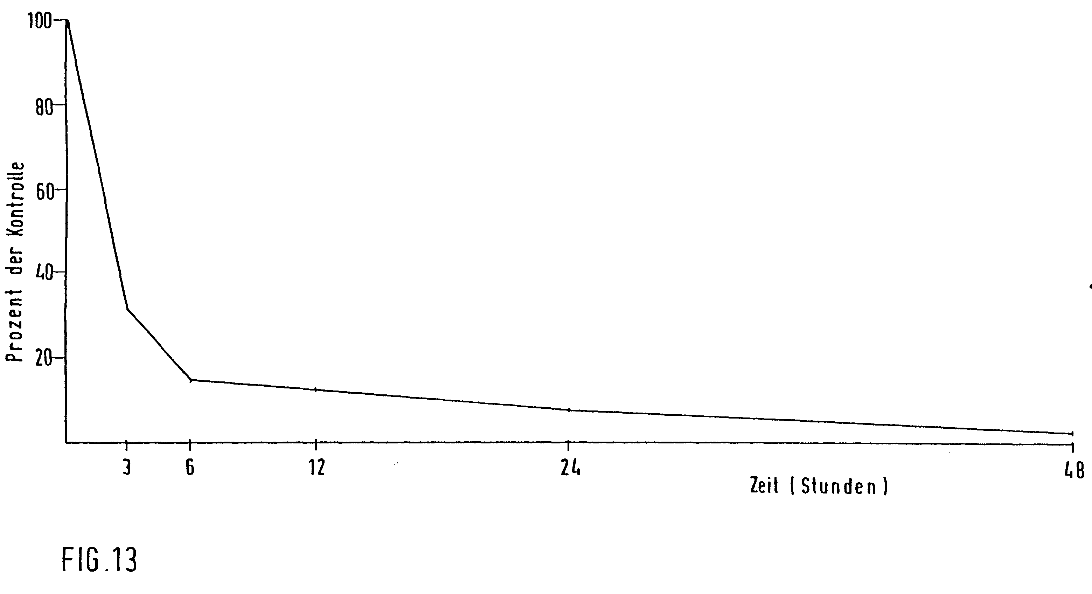

Fig. 13 zeigt, daß der zytotoxische Effekt des erfindungsgemäßen Peptids P3 [10⁻⁵M] auf asynchrone K562-Zellen, die in RPMI-Zellkulturmedium mit 10 % FCS inkubiert wurden, zeitabhängig ist. Die Ergebnisse wurden aufgetragen als lebende, mit P3 behandelte Zellen als Prozent der lebenden, mit P3 nicht behandelten Zellen. Die Zahl der lebenden Zellen wurde ermittelt, indem man mindestens 200 Zellen mit der Trypanblau-Methode zu jedem angegebenen Zeitpunkt auszählte.

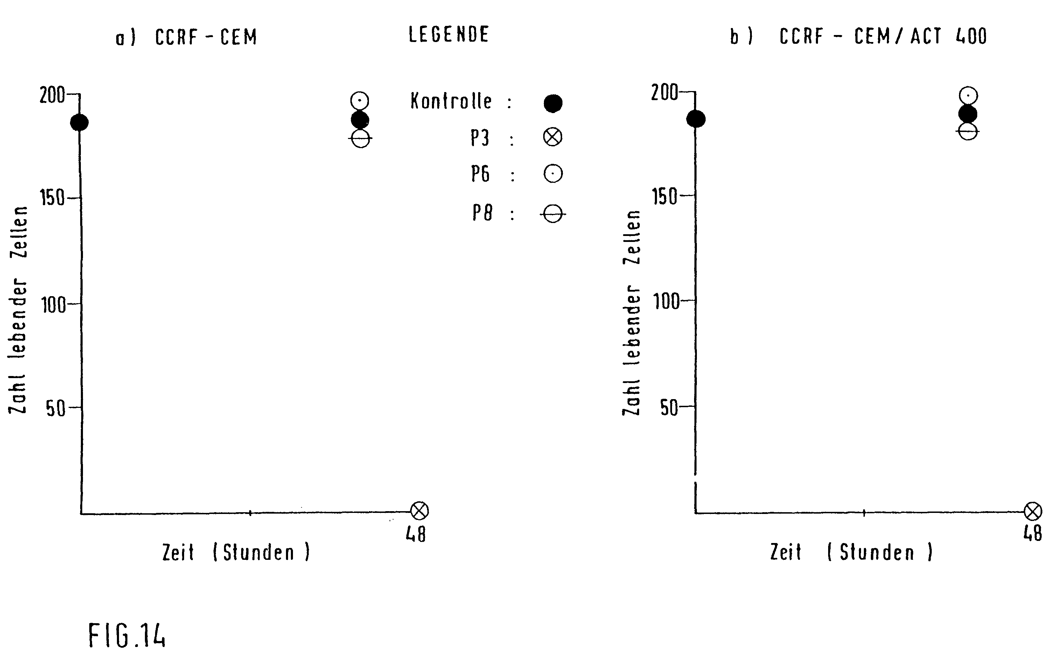

Fig. 14 zeigt, daß das erfindungsgemäße Peptid P3 [10⁻⁵M] zytotoxisch auf asynchrone CCRF-CEMsensitiv- und CCRF-CEM/ACT 400ᵣₑₛᵢₛₜₑₙₜ-Zellen wirkt, die in RPMI-Zellkulturmedium mit 10 % FCS inkubiert wurden.

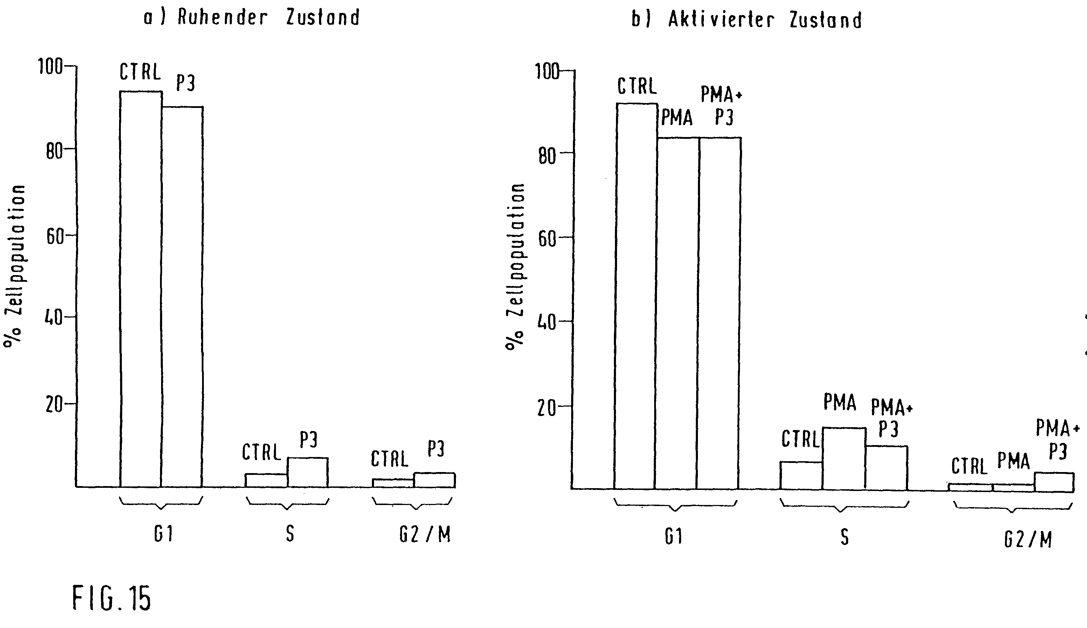

Fig. 15 zeigt, daß das erfindungsgemäße Peptid P3 [10⁻⁵M] keinen Effekt auf normale humane mononukleäre Zellen des peripheren Blutes hat, unabhängig davon, ob sich diese in einem ruhenden (a) oder aktivierten (b) Zustand befinden. Entsprechend weisen die mit P3 behandelten Zellen auch keine morphologischen Veränderungen auf. Die Inkubation der Zellen mit P3 erfolgte für einen Zeitraum von 24 Stunden.

Tab. 1 zeigt, daß die erfindungsgemäßen Peptide P5 und P6, je in einer Konzentration von 10⁻⁵M, die S-Phase von MCF-7-Zellen vermindern, die durch IGF-1[10⁻⁸M] bzw. durch Insulin [10⁻⁶M] stimuliert wurden. Das Peptid Penetratin [10⁻⁵M] hat keinen Effekt in diesem Assay, so wie erwartet.

Tab. 2 zeigt, daß die erfindungsgemäßen Peptide P5 und P6, je in einer Konzentration von 5 x 10⁻⁵M, zu einer Zunahme der G1-Phase und einer Abnahme der S-Phase von SAOS-2-Zellen führen, die durch 10 % FCS stimuliert wurden.

Insgesamt läßt sich anhand der beiliegenden Figuren 1-15 sowie der Tabellen 1-2 feststellen, daß die erfindungsgemäßen Peptide P3, P5, P6, P7s oder P8 über die Eigenschaft verfügen, das Fortschreiten des Zellzyklus und damit die Zellteilung der MCF-7-Mammakarzinom (=Brustkrebs)-Zellen, der SAOS-2-Osteosarkom (=Knochenkrebs)-Zellen oder von Leukämie (=Blutkrebs)-Zellen (K562, CCRF-CEMₛₑₙₛᵢₜᵢᵥ, CCRF-CEM/ACT 400ᵣₑₛᵢₛₜₑₙₜ) unter bestimmten Zellkultur-Bedingungen nachhaltig zu bremsen, und zwar allein oder/und in Kombination. Die Daten, die für IGF-1[10⁻⁸M] hier gezeigt werden, gelten auch für Insulin [10⁻⁶M].

Diese Peptide haben damit das Potential, auch in vivo im Menschen als wirksame antineoplastische Substanzen gegen das Krebswachstum zu agieren.

Die Erfindung wird nun weiter durch die Beispiele erläutert:

### Beispiele

Die eine Versuchsanordnung beinhaltet Versuche mit der humanen, intaktes Retinoblastomprotein enthaltenden Mammakarzinom-Zell-Linie MCF-7 bzw. der humanen, defektes Retinoblastomprotein enthaltenden Osteosarkom-Zell-Linie SAOS-2. Man sät die Zellen zuerst in 12-well-Platten in einer Zelldichte von 100.000 (= Hunderttausend) Zellen/well/ml RPMI oder DMEM/10% PCS) aus, läßt sie 24 Stunden adhärieren, hungert sie anschließend 3 Tage in DMEM ohne FCS mit dem Zweck, die Zellen in der G0/G1-Phase zu synchronisieren, und stimuliert sie dann entweder mit 10% FCS oder 10⁻⁸ M IGF-1 oder 10⁻⁶ M Insulin für 24 Stunden, wobei der Effekt der jeweils zugegebenen Peptide auf die jeweilige Stimulation, die ein Maß für die Zellteilungsrate ist, untersucht wird. Die Kontrollgruppe (G0/G1-synchronisierte Zellen) wird zum Zeitpunkt 0 nach der dreitägigen Hungerphase fixiert, die restlichen Zellen (+/- Peptid) werden nach 24 Stunden fixiert. Daraufhin werden die Zellen zwecks Zellzyklusanalyse im FACS analysiert. Analoge Methoden finden sich beispielsweise bei R. Fahraeus et al., Current Biology, 1996, Vol. 6, No. 1, pp 84-91 sowie bei L. Zhu et al., Genes & Development, 1993, Vol. 7, pp. 1111-1125.

Die zweite Versuchsanordnung betrifft die Leukämie-Zell-Linien K562 und CCRF-CEM und CCRF-CEM/ACT400. Hierbei wurden jeweils 100.000 (= Hunderttausend) asynchrone Zellen/well/ml RPMI/10% FCS für 48 Stunden mit den jeweiligen Peptiden in 6-well-Platten inkubiert. Als Meßgröße gilt die Anzahl der mit Hilfe der Trypanblaufärbung (L.D. Attardi et al., The EMBO Journal, 1996, Vol. 15, No. 14, pp. 3693-3701 und M.K. Reeder & H.C. Isom, Cell Growth & Differentiation, 1996, Vol. 7, pp. 449-460) nach 48 Stunden Inkubationszeit festgestellten lebenden Zellen/200 ausgezählte Zellen. Je weniger lebende Zellen, desto zytotoxischer das Peptid und damit umso effektiver.

Die Ergebnisse der Beispiele sind in den Figuren 1 bis 15 sowie in den Tabellen 1 und 2 gezeigt.

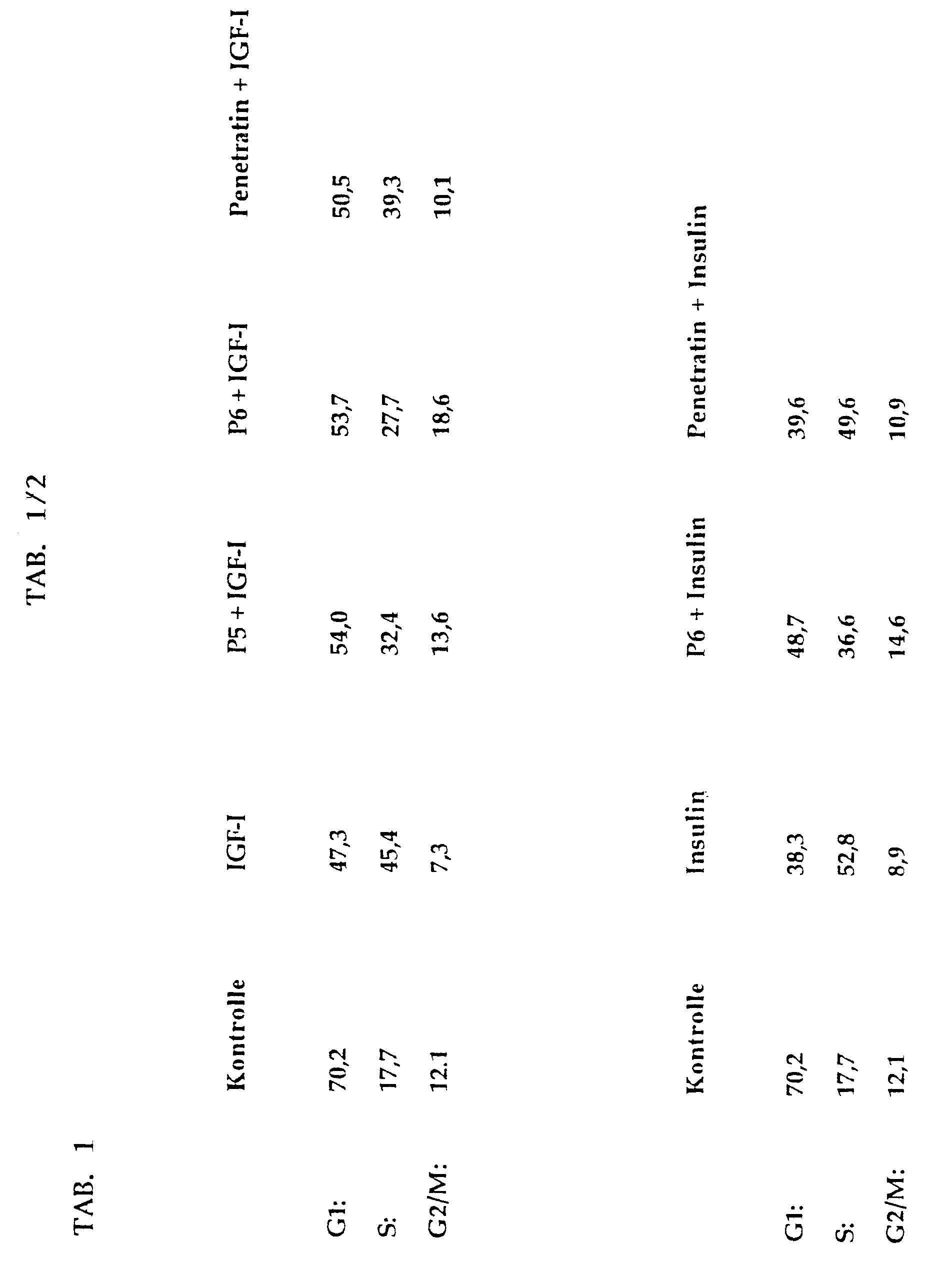

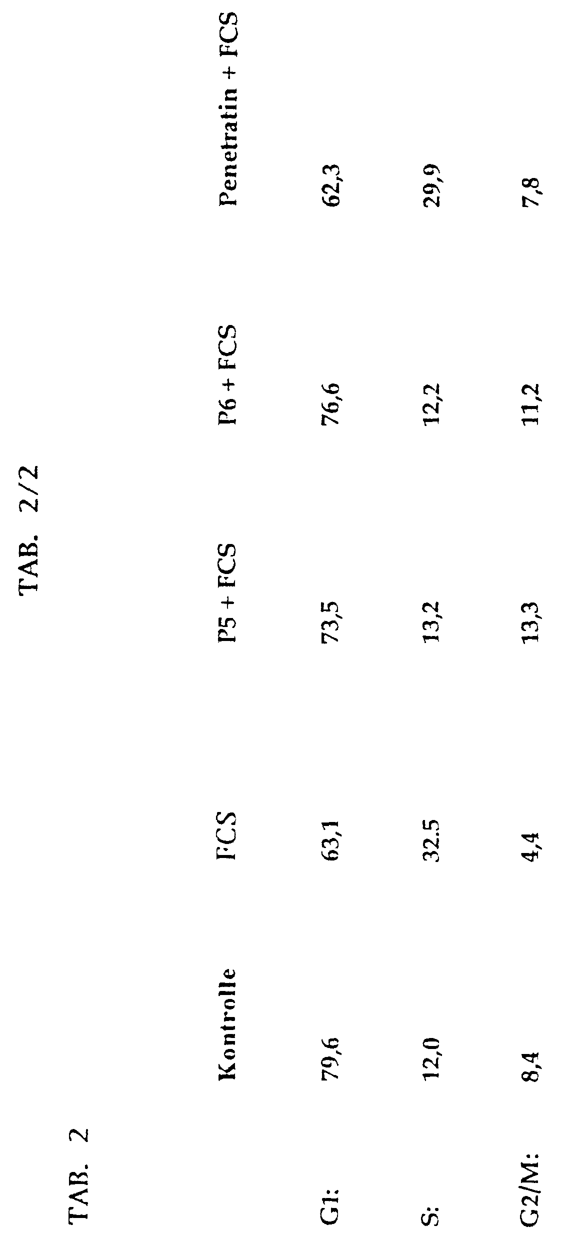

## Patentansprüche

1. Antiproliferatives Peptid AB oder BA, bestehend aus:
- einem antiproliferativen Teil (A), der ein Fragment eines Tumorsuppressorproteins oder eine dazu hydropathisch homologe Peptidsequenz umfaßt, und der die Eigenschaft besitzt, an ein Wachstumsfaktor-Segment oder an ein Wachstumsfaktorrezeptor-Segment zu binden,
- einem Teil (B), der als Kofaktor die Eigenschaft besitzt, den Teil (A) zu stabilisieren und vor einem proteolytischen Abbau durch Proteasen zu schützen, und der eine nukleäre Lokalisationssequenz (NLS) ist, die ausgewählt ist unter
a) PKKKRKV
b) RQIKIWFQNRRMKWKK
c) einer bipartiten NLS
d) der RNP A1 NLS.

2. Peptid nach Anspruch 1,
wobei der antiproliferative Teil (A) zu einem Wachstumsfaktor-Segment oder einem Wachstumsfaktorrezeptor-Segment hydropathisch komplementär ist.

3. Peptid nach Anspruch 1 oder 2,
wobei der antiproliferative Teil (A) ein von dem Retinoblastomprotein (RB1) abgeleitetes Tumorsuppressorprotein-Fragment ist.

4. Peptid nach einem der Ansprüche 1 bis 3,
wobei der Teil (A) die Aminosäuresequenz FYKK oder eine in mindestens zwei Positionen dazu hydropathisch homologe Aminosäuresequenz umfaßt.

5. Peptid nach Anspruch 4,
wobei der Teil (A) die Aminosäuresequenz LFYKKV oder eine in mindestens zwei Positionen dazu hydropathisch homologe Aminosäuresequenz umfaßt.

6. Peptid nach einem der vorhergehenden Ansprüche,
wobei das Wachstumsfaktor-Segment oder das Wachstumsfaktorrezeptor-Segment eine Aminosäuresequenz mit dem folgenden hydropathischen Profil umfaßt:
hydrophobe Aminosäure - X - hydrophobe Aminosäure - X - hydrophile Aminosäure,
worin X eine beliebige Aminosäure darstellt.

7. Peptid nach Anspruch 6,
wobei das Wachstumsfaktor-Segment oder das Wachstumsfaktorrezeptor-Segment die Aminosäuresequenz LXCXE oder FVCGD umfaßt,
wobei X eine beliebige Aminosäure darstellt.

8. Peptid nach Anspruch 7,
wobei die Aminosäuresequenz LXCXE von Insulin abgeleitet ist.

9. Peptid nach einem der vorhergehenden Ansprüche,
wobei das Peptid ausgewählt ist unter
a) LFYKKVPKKKRKV
b) (all-D) LFYKKVPKKKRKV
c) LFYKKVRQIKIWFQNRRMKWKK
d) (all-D) LFYKKVRQIKIWFQNRRMKWKK
e) KVLYFKRQIKIWFQNRRMKWKK
f) (all-D) KVLYFKRQIKIWFQNRRMKWKK.

10. Peptidnukleinsäure, deren Struktur homolog ist zu der einer Nukleinsäure, die für ein Peptid nach einem der vorhergehenden Ansprüche entsprechend dem genetischen Code kodiert.

11. Peptidnukleinsäure nach Anspruch 10,
wobei die Nukleinsäure die folgende Sequenz umfaßt:
a) 5'- UUC UAC AAG AAG - 3'    (D0) oder eine für das gleiche Peptid kodierende Nukleinsäure,
b) in der ein oder mehrere Nukleotide ersetzt sind, oder
c) die mit der Nukleinsäure (D0) hybridisiert, oder
d) die mit der Nukleinsäure (D0) über den degenerierten genetischen Code verwandt ist.

12. Peptidnukleinsäure nach Anspruch 10, wobei die Nukleinsäure die folgende Sequenz umfaßt:
a) 5'- CUU UUC UAC AAG AAG GUU - 3'     (D1) oder eine für das gleiche Peptid kodierende Nukleinsäure, in der
b) ein oder mehrere Nukleotide ersetzt sind, oder
c) die mit der Nukleinsäure (D1) hybridisiert, oder
d) die mit der Nukleinsäure (D1) über den degenerierten genetischen Code verwandt ist.

13. Pharmazeutische Zusammensetzung, welche ein oder mehrere Peptide nach einem der Ansprüche 1 bis 9 enthält.

14. Pharmazeutische Zusammensetzung nach Anspruch 13 in Form von Salben, Lösungen, Dispersionen, Emulsionen, Aerosolen, Schäumen, teilchenförmigen Mitteln, Pillen, Pastillen, Tabletten, Dragees oder Kapseln.

15. Pharmazeutische Zusammensetzung nach Anspruch 13,
bei der die teilchenförmigen Mittel ausgewählt werden unter Granulaten, Agglomeraten, Puder, Mikroperlen und Adsorbaten.

16. Verwendung eines antiproliferativen Peptids AB oder BA, bestehend aus:
- einem antiproliferativen Teil (A), der ein Fragment eines Tumorsuppressorproteins oder eine dazu hydropathisch homologe Peptidsequenz umfaßt, und der die Eigenschaft besitzt, an ein Wachstumsfaktor-Segment oder an ein Wachstumsfaktorrezeptor-Segment zu binden,
- einem Teil (B), der als Kofaktor die Eigenschaft besitzt, den Teil (A) zu stabilisieren und vor einem proteolytischen Abbau durch Proteasen zu schützen,
zur Herstellung eines Mittels zur Diagnose und/oder Therapie von Erkrankungen, die mit einer gesteigerten Proliferation bzw. Hyperproliferation von Zellen einhergehen.

17. Verwendung nach Anspruch 16,
wobei der antiproliferative Teil (A) zu einem Wachstumsfaktor-Segment oder einem Wachstumsfaktorrezeptor-Segment hydropathisch komplementär ist.

18. Verwendung nach Anspruch 16 oder 17,
wobei der antiproliferative Teil (A) ein von dem Retinoblastomprotein (RB1) abgeleitetes Tumorsuppressorprotein-Fragment ist.

19. Verwendung nach einem der Ansprüche 16 bis 18,
wobei der Teil (A) die Aminosäuresequenz FYKK oder eine in mindestens zwei Positionen dazu hydropathisch homologe Aminosäuresequenz umfaßt.

20. Verwendung nach Anspruch 19,
wobei der Teil (A) die Aminosäuresequenz LFYKKV oder eine in mindestens zwei Positionen dazu hydropathisch homologe Aminosäuresequenz umfaßt.

21. Verwendung nach einem der Ansprüche 16 bis 20,
wobei der Teil (B) ausgewählt ist unter einem verzweigten Peptid, einem Polylysin-Core, D-Aminosäuren und einer nukleären Lokalisationssequenz (NLS).

22. Verwendung nach Anspruch 21,
wobei der Teil (B) ein Polylysin-Core ist, der ausgewählt ist unter
a) [GGG]₄[KRG]₂KG
b) [GGG]₄[K]₂KG
c) [GGG]₄[lKdRG]₂lKG.

23. Verwendung nach Anspruch 21,
wobei der Teil (B) eine NLS ist, die ausgewählt ist unter
a) PKKKRKV
b) RQIKIWFQNRRMKWKK
c) einer bipartiten NLS
d) der RNP A1 NLS.

24. Verwendung nach einem der Ansprüche 16 bis 23,
wobei das Wachstumsfaktor-Segment oder das Wachstumsfaktorrezeptor-Segment eine Aminosäuresequenz mit dem folgenden hydropathischen Profil umfaßt:
hydrophobe Aminosäure - X - hydrophobe Aminosäure - X - hydrophile Aminosäure,
worin X eine beliebige Aminosäure darstellt.

25. Verwendung nach Anspruch 24,
wobei das Wachstumsfaktor-Segment oder das Wachstumsfaktorrezeptor-Segment die Aminosäuresequenz LXCXE oder FVCGD umfaßt,
wobei X eine beliebige Aminosäure darstellt.

26. Verwendung nach Anspruch 25,
wobei die Aminosäuresequenz LXCXE von Insulin abgeleitet ist.

27. Verwendung nach einem der Ansprüche 16 bis 26,
wobei das Peptid ausgewählt ist unter
a) [LFYKKVGGG]₄[KRG]₂KG
b) [dLdFdYdKdKdVGGG]₄[lKdRG]₂lKG
c) [dLdFdYdKdKdVGGG]₄[lK]₂lKG
d) LFYKKVPKKKRKV
e) (all-D) LFYKKVPKKKRKV
f) LFYKKVRQIKIWFQNRRMKWKK
g) (all-D) LFYKKVRQIKIWFQNRRMKWKK
h) [dKdVdLdYdFdKGGG]₄[lKdRG]₂lKG
i) [dKdVdLdYdFdKGGG]₄[lK]₂lKG
j) KVLYFKRQIKIWFQNRRMKWKK
k) (all-D) KVLYFKRQIKIWFQNRRMKWKK.

28. Verwendung einer Nukleinsäure, die entsprechend dem genetischen Code für ein Peptid AB oder BA kodiert, das aus folgenden Teilen besteht:
- einem antiproliferativen Teil (A), der ein Fragment eines Tumorsuppressorproteins oder eine dazu hydropathisch homologe Peptidsequenz umfaßt, und der die Eigenschaft besitzt, an ein Wachstumsfaktor-Segment oder an ein Wachstumsfaktorrezeptor-Segment zu binden,
- einem Teil (B), der als Kofaktor die Eigenschaft besitzt, den Teil (A) zu stabilisieren und vor einem proteolytischen Abbau durch Proteasen zu schützen,
zur Herstellung eines Mittels zur Diagnose und/oder Therapie von Erkrankungen, die mit einer gesteigerten Proliferation bzw. Hyperproliferation von Zellen einhergehen.

29. Verwendung einer Nukleinsäure nach Anspruch 28,
wobei die Nukleinsäure die folgende Sequenz umfaßt:
a) 5'- UUC UAC AAG AAG - 3'    (D0) oder eine für das gleiche Peptid kodierende Nukleinsäure,
b) in der ein oder mehrere Nukleotide ersetzt sind, oder
c) die mit der Nukleinsäure (D0) hybridisiert, oder
d) die mit der Nukleinsäure (D0) über den degenerierten genetischen Code verwandt ist.

30. Verwendung einer Nukleinsäure nach Anspruch 28,
wobei die Nukleinsäure die folgende Sequenz umfaßt:
a) 5'- CUU UUC UAC AAG AAG GUU - 3'          (D1) oder eine für das gleiche Peptid kodierende Nukleinsäure, in der
b) ein oder mehrere Nukleotide ersetzt sind, oder
c) die mit der Nukleinsäure (D1) hybridisiert, oder
d) die mit der Nukleinsäure (D1) über den degenerierten genetischen Code verwandt ist.

31. Verwendung einer Peptidnukleinsäure, deren Struktur homolog ist zu der einer Nukleinsäure gemäß einem der Ansprüche 28 bis 30.

32. Verwendung einer pharmazeutischen Zusammensetzung nach Anspruch 13 zur Herstellung eines Mittels zur Diagnose und/oder Therapie von hyperproliferativen Erkrankungen.

33. Verwendung nach einem der Ansprüche 16 bis 32, wobei die Erkrankung ein benigner Tumor, ein maligner Tumor oder Atherosklerose ist.

## Claims

1. An antiproliferative peptide AB or BA consisting of:
- an antiproliferative component (A) comprising a fragment of a tumor suppressor protein or a peptide sequence hydropathically homologous thereto, having the property of binding to a growth factor segment or to a growth factor receptor segment;
- a component (B) that is a cofactor having the property of stabilizing component (A) and protecting component (A) from proteolytic degradation by proteases, and that is a nuclear localization sequence (NLS) selected from the group consisting of
a) PKKKRKV;
b) RQIKIWFQNRRMKWKK;
c) a bipartite NLS;
d) the RNP A1 NLS.

2. The peptide according to claim 1, wherein component (A) is hydropathically complementary to a growth factor segment or a growth factor receptor segment.

3. The peptide according to claim 1 or 2, wherein component (A) is a tumor suppressor protein fragment derived from retinoblastoma protein (RB1).

4. The peptide according to any of the claims 1 to 3, wherein component (A) comprises the amino acid sequence FYKK or an amino acid sequence that is hydropathically homologous thereto in at least two positions.

5. The peptide according to claim 4, wherein component (A) comprises the amino acid sequence LFYKKV or an amino acid sequence that is hydropathically homologous thereto in at least two positions.

6. The peptide according to any of the preceding claims, whereby the growth factor segment or the growth factor receptor segment comprises an amino acid sequence with the following hydropathic profile: hydrophobic amino acid - X - hydrophobic amino acid - X - hydrophilic amino acid, wherein X represents any amino acid.

7. The peptide according to claim 6, whereby the growth factor segment or the growth factor receptor segment comprises the amino acid sequence LXCXE or FVCGD, wherein X represents any amino acid.

8. The peptide according to claim 7, whereby the amino acid sequence LXCXE is derived from insulin.

9. The peptide according to any of the preceding claims, whereby the peptide is selected from the group consisting of
a) LFYKKVPKKKRKV;
b) (all-D) LFYKKVPKKKRKV;
c) LFYKKVRQIKIWFQNRRMKWKK;
d) (all-D) LFYKKVRQIKIWFQNRRMKWKK;
e) KVLYFKRQIKIWFQNRRMKWKK;
f) (all-D) KVLYFKRQIKIWFQNRRMKWKK.

10. A peptide nucleic acid that is structurally homologous to a nucleic acid encoding a peptide according to any of the preceding claims based on the genetic code.

11. The peptide nucleic acid according to claim 10, whereby the nucleic acid comprises the following sequence:
a) 5′ - UUC UAC AAG AAG - 3′ (D0) or a nucleic acid encoding the same peptide
b) in which one or more nucleotides have been exchanged;
c) that hybridizes with nucleic acid (D0), or
d) that is related to nucleic acid (D0) through the degeneracy of the genetic code.

12. The peptide nucleic acid according to claim 10, whereby the nucleic acid comprises the following sequence:
a) 5′ - CUU UUC UAC AAG AAG GUU- 3′ (D1) or a nucleic acid encoding the same peptide
b) in which one or more nucleotides have been exchanged;
c) that hybridizes with nucleic acid (D1), or
d) that is related to nucleic acid (D1) through the degeneracy of the genetic code.

13. A pharmaceutical composition containing one or more peptides according to any of the claims 1 to 9.

14. The pharmaceutical composition according to claim 13 in the form of ointments, solutions, dispersions, emulsions, aerosols, foams, particles, pills, pastilles, tablets, dragees or capsules.

15. The pharmaceutical composition according to claim 13, wherein the particles are selected from the group consisting of granulates, agglomerates, powders, micropearls and adsorbates.

16. Use of an antiproliferative peptide AB or BA consisting of:
- an antiproliferative component (A) comprising a fragment of a tumor suppressor protein or a peptide sequence hydropathically homologous thereto having the property of binding to a growth factor segment or to a growth factor receptor segment;
- a component (B) that is a cofactor having the properties of stabilizing component (A) and protecting component (A) from proteolytic degradation by proteases,
in the preparation of an agent for the diagnosis and/or treatment of diseases associated with increased cellular proliferation or hyperproliferation, respectively.

17. The use according to claim 16, whereby the antiproliferative component (A) is hydropathically complementary to a growth factor segment or a growth factor receptor segment.

18. The use according to claim 16 or 17, whereby the antiproliferative component (A) is a tumor suppressor protein fragment derived from retinoblastoma protein (RB1).

19. The use according to any of the claims 16 to 18, whereby component (A) comprises the amino acid sequence FYKK or an amino acid sequence that is hydropathically homologous thereto in at least two positions.

20. The use according to claim 19, whereby component (A) comprises the amino acid sequence LFYKKV or an amino acid sequence that is hydropathically homologous thereto in at least two positions.

21. The use according to any of the claims 16 to 20, whereby component (B) is selected from the group consisting of a branched peptide, a polylysine core, D-amino acids and a nuclear localization sequence (NLS).

22. The use according to claim 21, whereby component (B) is a polylysine core selected from the group consisting of
a) [GGG]₄[KRG]₂KG;
b) [GGG]₄[K]₂KG;
c) [GGG]₄[lKdRG]₂lKG.

23. The use according to claim 21, whereby component (B) is an NLS selected from the group consisting of
a) PKKKRKV;
b) RQIKIWFQNRRMKWKK;
c) a bipartite NLS;
d) the RNP A1 NLS.

24. The use according to any of the claims 16 to 23, whereby the growth factor segment or the growth factor receptor segment comprises an amino acid sequence with the following hydropathic profile: hydrophobic amino acid - X - hydrophobic amino acid - X - hydrophilic amino acid, wherein X represents any amino acid.

25. The use according to claim 24, whereby the growth factor segment or the growth factor receptor segment comprises the amino acid sequence LXCXE or FVCGD, wherein X represents any amino acid.

26. The use according to claim 25, whereby the amino acid sequence LXCXE is derived from insulin.

27. The use according to any of the claims 16 to 26, whereby the peptide is selected from the group consisting of
a) [LFYKKVGGG]₄[KRG]₂KG
b) [dLdFdYdKdKdVGGG]₄[lKdRG]₂lKG
c) [dLdFdYdKdKdVGGG]₄[lK]₂lKG
d) LFYKKVPKKKRKV
e) (all-D) LFYKKVPKKKRKV
f) LFYKKVRQIKIWFQNRRMKWKK
g) (all-D) LFYKKVRQIKIWFQNRRMKWKK
h) [dKdVdLdYdFdKGGG]₄[lKdRG]₂lKG
i) [dKdVdLdYdFdKGGG]₄[lK]₂lKG
j) KVLYFKRQIKIWFQNRRMKWKK
k) (all-D) KVLYFKRQIKIWFQNRRMKWKK.

28. Use of a nucleic acid encoding a peptide AB or BA according to the genetic code, whereby the peptide consists of the following components:
- an antiproliferative component (A) comprising a fragment of a tumor suppressor protein or a peptide sequence hydropathically homologous thereto having the property of binding to a growth factor segment or to a growth factor receptor segment;
- a component (B) that is a cofactor having the properties of stabilizing component (A) and protecting component (A) from proteolytic degradation by proteases,
in the preparation of an agent for the diagnosis and/or treatment of diseases associated with an increased cellular proliferation or hyperproliferation, respectively.

29. The use of a nucleic acid according to claim 28, whereby the nucleic acid comprises the following sequence:
a) 5′ - UUC UAC AAG AAG - 3′ (D0) or a nucleic acid encoding the same peptide
b) in which one or more nucleotides have been exchanged;
c) that hybridizes with nucleic acid (D0), or
d) that is related to nucleic acid (D0) through the degeneracy of the genetic code.

30. The use of a nucleic acid according to claim 28, whereby the nucleic acid comprises the following sequence:
a) 5′ - CUU UUC UAC AAG AAG GUU - 3′ (D1) or a nucleic acid encoding the same peptide
b) in which one or more nucleotides have been exchanged;
c) that hybridizes with nucleic acid (D1), or
d) that is related to nucleic acid (D1) through the degeneracy of the genetic code.

31. Use of a peptide nucleic acid that is structurally homologous to a nucleic acid according to any of the claims 28 to 30.

32. Use of a pharmaceutical composition according to claim 13 in the preparation of an agent for the diagnosis and/or treatment of hyperproliferative diseases.

33. The use according to any one of the claims 16 to 32,whereby the disease is a benign tumor, a malignant tumor or atherosclerosis.

## Revendications

1. Peptide antiprolifératif AB ou BA qui consiste:
- d'une partie antiproliférative (A) qui comprend un fragment d'une protéine suppresseur de tumeur ou une séquence peptidique qui en est hydropathiquement homologue et qui possède la qualité de se lier à un segment d'un facteur de croissance ou à un segment d'un récepteur pour un facteur de croissance,
- d'une partie (B) qui possède en tant que cofacteur la qualité de stabiliser la partie (A) et de la protéger contre une dégradation protéolytique par des protéases et qui est une séquence de localisation nucléaire (SLN) sélectionnée dans le groupe constitué par
a) PKKKRKV;
b) RQIKIWFQNRRMKWKK;
c) une SLN bipartite;
d) la SLN de la RNP A1.

2. Peptide selon la revendication 1, **caractérisé en ce que** la partie antiproliférative (A) est hydropathiquement complémentaire à un segment d'un facteur de croissance ou à un segment d'un récepteur pour un facteur de croissance.

3. Peptide selon la revendication 1 ou la revendication 2, **caractérisé en ce que** la partie antiproliférative (A) est un fragment de protéine suppresseur de tumeur qui provient de la protéine du rétinoblastome (RB1).

4. Peptide selon l'une quelconque des revendications 1 à 3, **caractérisé en ce que** la partie (A) comprend la séquence d'acides aminés FYKK ou une séquence d'acides aminés qui en est hydropathiquement homologue dans au moins deux positions.

5. Peptide selon la revendication 4, **caractérisé en ce que** la partie (A) comprend la séquence d'acides aminés LFYKKV ou une séquence d'acides aminés qui en est hydropathiquement homologue dans au moins deux positions.

6. Peptide selon l'une quelconque des revendications précédentes, **caractérisé en ce que** le segment du facteur de croissance ou le segment du récepteur pour le facteur de croissance comprend une séquence d'acides aminés ayant le profil hydropathique suivant: acide aminé hydrophobe - X - acide aminé hydrophobe - X - acide aminé hydrophile, **caractérisé en ce que** X représente un acide aminé quelconque.

7. Peptide selon la revendication 6, **caractérisé en ce que** le segment du facteur de croissance ou le segment du récepteur pour le facteur de croissance comprend la séquence d'acides aminés LXCXE ou FVCGD, **caractérisée en ce que** X représente un acide aminé quelconque.

8. Peptide selon la revendication 7, **caractérisé en ce que** la séquence d'acides aminés LXCXE provient de l'insuline.

9. Peptide selon l'une quelconque des revendications précédentes, **caractérisé en ce que** le peptide est sélectionné dans le groupe constitué par
a) LFYKKVPKKKRKV;
b) (all-D) LFYKKVPKKKRKV;
c) LFYKKVRQIKIWFQNRRMKWKK;
d) (all-D) LFYKKVRQIKIWFQNRRMKWKK;
e) KVLYFKRQIKIWFQNRRMKWKK;
f) (all-D) KVLYFKRQIKIWFQNRRMKWKK.

10. Peptide acide nucléique dont la structure est homologue à celle d'un acide nucléique qui code pour un peptide selon l'une quelconque des revendications précédentes suivant le code génétique.

11. Peptide acide nucléique selon la revendication 10, **caractérisé en ce que** l'acide nucléique comprend la séquence suivante:
a) 5′ - UUC UAC AAG AAG - 3′ (D0) ou un acide nucléique codant pour le même peptide,
b) dans lequel acide nucléique un ou plusieurs nucléotides sont substitués, ou
c) lequel acide nucléique hybridise avec l'acide nucléique (D0), ou
d) lequel acide nucléique est apparenté avec l'acide nucléique (D0) par le code génétique dégénéré.

12. Peptide acide nucléique selon la revendication 10, **caractérisé en ce que** l'acide nucléique comprend la séquence suivante:
a) 5′- CUU UUC UAC AAG AAG GUU -3′ (D1) ou un acide nucléique codant pour le même peptide,
b) dans lequel acide nucléique un ou plusieurs nucléotides sont substitués, ou
c) lequel acide nucléique hybridise avec l'acide nucléique (D1), ou
d) lequel acide nucléique est apparenté avec l'acide nucléique (D1) par le code génétique dégénéré.

13. Composition pharmacéutique qui contient un ou plusieurs peptides selon l'une quelconque des revendications 1 à 9.

14. Composition pharmacéutique selon la revendication 13 en tant que pommades, solutions, dispersions, émulsions, aérosols, mousses, moyens particulés, pilules, losanges, pastilles, dragées ou capsules.

15. Composition pharmacéutique selon la revendication 13, **caractérisée en ce que** les moyens particulés sont sélectionnés dans le groupe constitué par granulats, agglomérés, poudres, microperles et adsorbats.

16. Utilisation d'un peptide antiprolifératif AB ou BA qui consiste:
- d'une partie antiproliférative (A) qui comprend un fragment d'une protéine suppresseur de tumeur ou une séquence peptidique qui en est hydropathiquement homologue et qui possède la qualité de se lier à un segment d'un facteur de croissance ou à un segment d'un récepteur pour un facteur de croissance,
- d'une partie (B) qui possède en tant que cofacteur la qualité de stabiliser la partie (A) et de la protéger contre une dégradation protéolytique par des protéases,
dans la préparation d'un moyen pour le diagnostic et/ou le traitement des maladies qui se caractérisent par une prolifération élevée respectivement une hyperprolifération des cellules.

17. Utilisation selon la revendication 16, **caractérisée en ce que** la partie antiproliférative (A) est hydropathiquement complémentaire à un segment d'un facteur de croissance ou à un segment d'un récepteur pour un facteur de croissance.

18. Utilisation selon la revendication 16 ou 17, **caractérisée en ce que** la partie antiproliférative (A) est un fragment de protéine suppresseur de tumeur qui provient de la protéine du rétinoblastome (RB1).

19. Utilisation selon l'une quelconque des revendications 16 à 18, **caractérisée en ce que** la partie (A) comprend la séquence d'acides aminés FYKK ou une séquence d'acides aminés qui en est hydropathiquement homologue dans au moins deux positions.

20. Utilisation selon la revendication 19,
**caractérisée en ce que** la partie (A) comprend la séquence d'acides aminés LFYKKV ou une séquence d'acides aminés qui en est hydropathiquement homologue dans au moins deux positions.

21. Utilisation selon l'une quelconque des revendications 16 à 20, **caractérisée en ce que** la partie (B) est sélectionnée dans le groupe constitué par un peptide ramifié, un polylysine-core, des D-acides aminés et une séquence de localisation nucléaire (SLN).

22. Utilisation selon la revendication 21,
**caractérisée en ce que** la partie (B) est un polylysine-core sélectionné dans le groupe constitué par
a) [GGG]₄[KRG]₂KG;
b) [GGG]₄[K]₂KG;
c) [GGG]₄[lKdRG]₂lKG.

23. Utilisation selon la revendication 21,
**caractérisée en ce que** la partie (B) est une SLN sélectionnée dans le groupe constitué par
a) PKKKRKV;
b) RQIKIWFQNRRMKWKK;
c) une SLN bipartite;
d) la SLN de la RNP A1.

24. Utilisation selon l'une quelconque des revendications 16 à 23,
**caractérisée en ce que** le segment du facteur de croissance ou le segment du récepteur pour le facteur de croissance comprend une séquence d'acides aminés ayant le profil hydropathique suivant: acide aminé hydrophobe - X - acide aminé hydrophobe - X - acide aminé hydrophile, **caractérisé en ce que** X représente un acide aminé quelconque.

25. Utilisation selon la revendication 24, **caractérisée en ce que** le segment du facteur de croissance ou le segment du récepteur pour le facteur de croissance comprend la séquence d'acides aminés LXCXE ou FVCGD, **caractérisée en ce que** X représente un acide aminé quelconque.

26. Utilisation selon la revendication 25, **caractérisée en ce que** la séquence d'acides aminés LXCXE provient de l'insuline.

27. Utilisation selon l'une quelconque des revendications 16 à 26,
**caractérisée en ce que** le peptide est sélectionné dans le groupe constitué par
a) [LFYKKVGGG]₄[KRG]₂KG
b) [dLdFdYdKdKdVGGG]₄[lKdRG]₂lKG
c) [dLdFdYdKdKdVGGG]₄[lK]₂lKG
d) LFYKKVPKKKRKV
e) (all-D) LFYKKVPKKKRKV
f) LFYKKVRQIKIWFQNRRMKWKK
g) (all-D) LFYKKVRQIKIWFQNRRMKWKK
h) [dKdVdLdYdFdKGGG]₄[lKdRG]₂lKG
i) [dKdVdLdYdFdKGGG]₄[lK]₂lKG
j) KVLYFKRQIKIWFQNRRMKWKK
k) (all-D) KVLYFKRQIKIWFQNRRMKWKK.

28. Utilisation d'un acide nucléique qui code selon le code génétique pour un peptide AB ou BA consistant des parties suivantes:
- une partie antiproliférative (A) qui comprend un fragment d'une protéine suppresseur de tumeur ou une séquence peptidique qui en est hydropathiquement homologue et qui possède la qualité de se lier à un segment d'un facteur de croissance ou à un segment d'un récepteur pour un facteur de croissance,
- une partie (B) qui possède en tant que cofacteur la qualité de stabiliser la partie (A) et de la protéger contre une dégradation protéolytique par des protéases,
dans la préparation d'un moyen pour le diagnostic et/ou le traitement des maladies qui se caractérisent par une prolifération élevée respectivement une hyperprolifération des cellules.

29. Utilisation d'un acide nucléique selon la revendication 28, **caractérisée en ce que** l'acide nucléique comprend la séquence suivante:
a) 5′- UUC UAC AAG AAG -3′ (D0) ou un acide nucléique codant pour le même peptide,
b) dans lequel acide nucléique un ou plusieurs nucléotides sont substitués, ou
c) lequel acide nucléique hybridise avec l'acide nucléique (D0), ou
d) lequel acide nucléique est apparenté avec l'acide nucléique (D0) par le code génétique dégénéré.

30. Utilisation d'un acide nucléique selon la revendication 28, **caractérisée en ce que** l'acide nucléique comprend la séquence suivante:
a) 5'- CUU UUC UAC AAG AAG GUU -3' (D1) ou un acide nucléique codant pour le même peptide,
b) dans lequel acide nucléique un ou plusieurs nucléotides sont substitués, ou
c) lequel acide nucléique hybridise avec l'acide nucléique (D1), ou
d) lequel acide nucléique est apparenté avec l'acide nucléique (D1) par le code génétique dégénéré.

31. Utilisation d'un peptide acide nucléique dont la structure est homologue à celle d'un acide nucléique selon l'une quelconque des revendications 28 à 30.

32. Utilisation d'une composition pharmacéutique selon la revendication 13 dans la préparation d'un moyen pour le diagnostic et/ou le traitement des maladies hyperprolifératives.

33. Utilisation selon l'une quelconque des revendications 16 à 32, **caractérisée en ce que** la maladie est une tumeur bénigne, une tumeur maligne ou athérosclérose.
